(19) Europäisches Patentamt
European Patent Office
Office européen des brevets

(11) Publication number: **0 180 964**
**B1**

(12) # EUROPEAN PATENT SPECIFICATION

(45) Date of publication of patent specification: **07.03.90**

(51) Int. Cl.$^5$: **C 12 N 15/00,** C 07 H 21/04, C 12 N 1/20, C 12 N 9/08 // C12R1/19

(21) Application number: **85114073.1**

(22) Date of filing: **05.11.85**

(54) **Recombinant DNA, microorganism transformed therewith, and process for producing human Cu-Zn superoxide dismutase using the transformed microorganism.**

(30) Priority: **06.11.84 JP 232395/84**

(43) Date of publication of application:
**14.05.86 Bulletin 86/20**

(45) Publication of the grant of the patent:
**07.03.90 Bulletin 90/10**

(84) Designated Contracting States:
**DE SE**

(56) References cited:
**EP-A-0 138 111**

**PROCEEDINGS OF THE NATIONAL ACADEMY OF SCIENCES OF THE UNITED STATES OF AMERICA, vol. 79, no. 9, May 1982 (Baltimore, USA) J. LIEMAN-HURWITZ et al. "Human cytoplasmic superoxide dismutase cDNA clone: A probe for studying the molecular biology of Down Syndrome" pages 2808-2811**

(73) Proprietor: **UBE INDUSTRIES LIMITED**
**12-32, 1-chome, Nishihoncho**
**Ube-shi, Yamaguchi-ken (JP)**

(72) Inventor: **Hiromi, Kumahara c/o Ube Research Laboratory**
**Ube Industries, Ltd. 1978-5, Oaza Kogushi**
**Ube-shi Yamaguchi-ken (JP)**
Inventor: **Masao, Maruyama c/o Ube Research Laboratory**
**Ube Industries, Ltd. 1978-5, Oaza Kogushi**
**Ube-shi Yamaguchi-ken (JP)**
Inventor: **Shigeharu, Anpeiji c/o Ube Research Laboratory**
**Ube Industries, Ltd. 1978-5, Oaza Kogushi**
**Ube-shi Yamaguchi-ken (JP)**
Inventor: **Takuji, Owa c/o Ube Research Laboratory**
**Ube Industries, Ltd. 1978-5, Oaza Kogushi**
**Ube-shi Yamaguchi-ken (JP)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 180 964 B1

(56) References cited:
BIOCHEMICAL AND BIOPHYSICAL RESEARCH COMMUNICATIONS, vol. 46, no. 2, 1972 (New York, London) A. NAKAZAWA et al. "Stimulation of Colicin E1 Synthesis by Cyclic 3', 5'-Adenosine Monophosphate in Mitomycin C-Induced Escherichia coli" pages 1004-1010

Biochemistry 19,11 (1980) 2310-16

Nucl. Acids Res., Vol. 13, no. 6 (1985) p. 2017-34

**Description**

This invention relates to a novel recombinant DNA and its use. More specifically, this invention relates to a recombinant DNA having a human Cu-Zn superoxide dismutase structural gene downstream of a regulatory gene, and its use.

Superoxide dismutase ("SOD" for short) is an enzyme having the action of scavenging superoxide ($O_2^-$) in accordance with the following disproportionation reaction.

$$O_2^- + O_2^- + 2H^- \rightarrow \dot{O}_2 + H_2O_2$$

SOD can be classified into three types, copper-zinc type (Cu, Zn-SOD), manganese type (Mn-SOD) and iron type (Fe-SOD) according to the metals contained in the molecule. Cu, Zn-SOD exists mainly in the cytoplasms of eukaryotes such as humans or animals, and Mn-SOD exists in mitochondria. Procaryotes such as bacteria, on the other hand, have both Mn-SOD and Fe-SOD within the cells.

It is McCord (Science, *185,* 529—531, 1974) who first discovered that SOD has an anti-inflammatory action. He used SOD purified from bovine erythrocytes in his work. The anti-inflammatory action of SOD derived from bacteria is also known (Japanese Laid-Open Patent Publication No. 16685/1983). These SODs are heterogenous proteins for humans, and involve a danger of causing undesirable immune responses. It is preferred to use human SOD as a medicine for humans. Much information has already been available on the method of preparing human SOD from human cells or tissues (see, for example, Japanese Laid-Open Patent Publications Nos. 141288/1982, 155991/1982, and 91881/1984).

EP—A—0 138 111 teaches the expression under the control of the hybrid "tac" promotor in cells of *E. coli* as well as the GAP promotor in yeast cells. This document, however, primarily differs altogether in technical concept from the present invention for the reason that this document is designed to check the influence on SOD output by changing in various ways the linker (or adaptor) between the human SOD structural gene and the ribosomal binding site of the promotor.

The said "tac" promotor of EP—A—0 138 111 does not have positive regulatory sites and because of this, it differs altogether from the regulatory gene (1) in the recombinant DNA of the present invention. Added to this, the recombinant DNA of the "tac" promotor and the human SOD structural gene is insignificantly small in SOD output.

As mentioned in this citation, page 24, lines 13 to 16, on the other hand, the GAL1/GAL10 regulatory gene is controlled by the GAL4 gene expression product.

Therefore, the positive regulation site in the regulatory gene (1) of the present invention is regulated (controlled) by a complex of cyclic AMP and cyclic AMP receptor protein.

Document I aims at increases in human SOD output by changing the linker in various ways with a focus placed on the linker region between the human SOD structural gene and the promotor, for which the present invention succeeded in markedly increasing SOD output by combination of the human SOD structural gene with a regulatory gene having a specified regulatory site.

In the citation "Biochemistry 19, 11 (1980), pages 2310—16" the whole amino acid sequence of SOD is described. However, the amino acid sequence of SOD described in this citation differs from the amino acid sequence of SOD which is predicted from cDNA in the present invention in the amino acid residues at six sites, i.e. 17th (Ile→Ser), 26th (Asn→Asp), 49th (Glu→Gla), 52nd (Asp→Asn), 53rd (Asn→Asp), and 98th (Ser→Val).

The prior methods, however, have not proved to be entirely suitable for industrial production because the raw material is difficult to obtain in large quantities, and the human placenta which is a relatively easily available raw material has a low SOD content.

A process utilizing recombinant DNA permits mass production and is industrially suitable.

To have the human SOD gene express within a microorganism and produce human SOD protein, the selection of a regulatory gene is important. Heretofore, regulatory genes called trp promoter, lac UV5 promoter, $P_L$ promoter, tac promoter, etc. have frequently been used. These regulatory genes undergo negative regulation, and as a regulation of gene expression in a microorganism, the existence of positive regulation is known. Furthermore, genes which undergo both negative regulation and positive regulation are known, and examples include lactose operon (lac), galactose operon (gal) and colicin E1 gene. In the regulatory genes of these genes, a region participating in the positive regulation exists upstream of a region participating in the negative regulation. In the negative regulation, usually a repressor binds to the operator region to repress expression. When the repression is released by an inducer, transcription by RNA polymerase bound to the promoter is started and expression takes place. It is known that the expression strikingly increases by the binding of a complex of cAMP and CRP (cAMP receptor protein) to a specific site (to be referred to as the positive regulation site) existing in the positive control region. No example, however, has been known in which such a regulatory gene having both positive and negative control regions was used in the expression of a human SOD gene.

Among these, the regulatory gene of colicin E1 gene is especially unique. The lac and gal genes undergo catabolite repression by glucose, and the expression in a medium containing glucose decreases. In contrast, colicin E1 gene shows a high expression in the presence of glucose (A. Nakazawa: Biochem. Biophys. Res. Commun., *46,* 1004—1010, 1972). It is known that two binding sites exist for the cAMP-CRP

complex which participates in the positive control (K. Shirabe et al.: Nucleic Acids Res., *13*, 4687—4698, 1985). The regulatory gene of colicin E1 gene is considered as suitable for mass-production of proteins.

With the foregoing background, the present inventors extensively worked on the production of human SOD by the recombinant DNA technology, and have consequently found that a human Cu, Zn-SOD gene can be efficiently expressed in a host by utilizing a regulatory gene having a positive regulation site.

It is an object of this invention to provide a recombinant DNA having a human Cu-Zn superoxide dismutase structural gene downstream of the Col E1 gene which is a regulatory gene having a positive regulation site.

Still another object of this invention is to provide a recombinant DNA which has a great ability to synthesize proteins even without imparting the function of increasing the protein synthesizing ability to a linker portion between the aforesaid structural gene and the regulatory gene.

A further object of this invention is to provide a process for producing the recombinant DNA of the invention.

A still further object of this invention is to provide a microorganism transformed with the recombinant DNA of the invention.

Additional objects and advantages of this invention will become apparent from the following description.

These objects and advantages of this invention are firstly achieved in accordance with this invention by a recombinant DNA comprising a regulatory gene having a positive regulation site and a human Cu-Zn superoxide dismutase structural cDNA, said positive regulation site being a specific site to bind a complex of cyclic AMP and cyclic AMP receptor protein, and the codon for the N-terminus alanine of said human Cu-Zn superoxide dismutase structural gene existing immediately after the translation initiation codon of the regulatory gene which is the regulatory gene of colicin E1 gene.

Figure 1 shows the base sequence of the regulatory gene region of colicin E1 gene. PB represents the RNA polymerase binding site and RS, the RNA polymerase recognition site. The bold arrow shows the transcription initiation point and the transcription direction. The broken underline shows the ribosome binding site. Met shows the position of the translation initiation codon. The two solid underlines show regions which participate in positive control. The restriction endonuclease *Dra*I site is also shown.

Figure 2 shows the base sequence of a human Cu-Zn SOD cDNA structural gene region obtained from mRNA of the placenta, and the amino acid sequence determined from the base sequence. The two underlined portions are regions with which two synthetic DNA probes used in colony hybridization hybridize.

Figure 3 shows the base sequence of a synthetic DNA fragment, and the base sequences of chemically synthesized oligonucleotides. In the synthetic DNA fragment obtained by ligating oligonucleotides, the 5′ terminus is the *Dra*I cleavage terminus, and the 3′ terminus is the *Taq*I cleavage terminus. The two asterisks show parts changed in base sequence from the base sequence shown in Figure 2, but the amino acid sequence remains unchanged.

Figure 4 shows the outline of the process for preparing the recombinant DNA pUBE2 of this invention.

Figure 5 shows an elution curve in SOD activity in column chromatography on ion exchange cellulose DE52. In Figure 5, the SOD activity is plotted in terms of percent inhibition of nitro blue tetrazolium (NBT) reduction. The straight line shows the concentration gradient of NaCl.

In the present invention, mRNA used as a template for synthesis of a human Cu, Zn-SOD cDNA is isolated from normal human tissues (liver, placenta, kidneys, etc.). Separation of RNA from the tissues may be effected by utilizing known methods such as the phenol-chloroform method, the guanidinium-hot phenol method and the guanidinium-cesium chloride method (T. Maniatis. et al., Molecular Cloning, 187—198, 1982, Cold Spring Harbor Laboratory). Then, by using oligo(dT) cellulose, poly(U) Sepharose, etc., mRNA having a poly(A) tail is separated.

The mRNA so obtained is an mRNA mixture containing human Cu, Zn-SOD mRNA which may be directly used in the synthesis of cDNA. First, a single-stranded cDNA is synthesized by using mRNA as a template and reverse transcriptase, and then a double-stranded cDNA is synthesized by using reverse transcriptase or DNA polymerase. cDNA is obtained in a form inserted into a suitable vector. For this purpose, the dG-dC or dA-dT homopolymer joining method (T. S. Nelson, Methods in Enzymology, *68*, 41, 1979, Academic Press Inc.) or the Okayama-Berg method (H. Okayama and P. Berg., Mol. Cell. Biol., 2, 161, 1082) can be utilized. When the amount of the desired mRNA is small in the mRNA mixture as in the present invention, the Okayama-Berg method which has a high efficiency is preferred. DNAs and host organisms required for this method can be obtained from Pharmacia P. L. Biochemicals, Inc. under Catalog No. 27-4750-01.

The recombinant DNA so obtained is introduced, for instance, into *E. coli* $\chi$ 1776 or DH 1 strain (B. Low, Proc. Natl. Acad. Sci., 160, 1968; M. Meselson and R. Yuan, Nature, *217*, 1110, 1968; D. Hanahan, J. Mol. Biol., 166, 557, 1983) to transform the host organism. Transformation may be carried out by a known method (K. Shigesada, Cell Engineering, Vol. 2, No. 3, 616, 1983) or a method similar to it. The transformants are screened by drug resistance such as ampicillin resistance. An oligonucleotide having a base sequence considered to correspond to the human Cu, Zn-SOD gene is chemically synthesized and labelled with $^{32}P$. By using the labelled oligonucleotide as a probe, those transformants which show a positive signal are selected by a known colony hybridization method (D. Hanahan, et. al., Methods in

Enzymology, *100,* 333, 1983). A plasmid DNA is isolated by a conventional method from these transformants. The base sequence of the cDNA portion is determined by the Maxam-Gilbert method (A. M. Maxam and W. Gilbert, Proc. Natl. Acad. Sci., *74,* 560, 1977) or the dideoxy method (J. Messing et al., Nucleic Acids Res., 9, 309, 1981), and the presence of the human Cu, Zn-SOD cDNA is confirmed. For the purpose of confirmation, the amino acid sequence of Cu, Zn-SOD isolated from human erythrocytes (J. R. Jabusch, Biochemistry, *19,* 2310, 1980) or the base sequence of cDNA obtained from mRNA which is isolated from an established cell line derived from patients with Down's syndrome (L. Sherman et al., Proc. Natl. Acad. Sci., *80,* 5465, 1983) may be referred to.

The resulting human Cu, Zn-SOD cDNA is joined to the downstream of a suitable regulatory gene. It should be noted however that an enzyme requiring $O_2^-$ is known to exist in a living organism (Yoshihiko Oyagi, Superoxide and Medicine, 58, 1981, Kyoritsu Shuppan K. K.). Accordingly, there is every possibility that the excessive production of SOD having the action of scavenging $O_2^-$ will cause a physiological trouble in the host organism. For the purpose of avoiding this possibility, it is preferred to grow the host cells in a condition where the expression of the gene is repressed, and thereafter to derepress and allow the gene to express in the host cells and produce large amounts of SOD.

The colicin E1 gene present on the plasmid Col E1 separated from *E. coli* is a gene which codes for the synthesis of colicin E1 protein, and in a normal condition, its expression is repressed by the binding of a represser protein to the operator. But when the colicin E1 gene is subjected to a treatment which damages DNA, such as ultraviolet irradiation, mitomycin C treatment or Nalidixic acid treatment, this repression is released, and the induction of the gene takes place whereby colicin E1 protein is produced in a large amount. This is generally called negative regulation. In addition, the colicin E1 gene has positive regulation (K. Shirabe et al., Biochemistry, Vol. 56, No. 8, 1082, 1984). Thus, the colicin E1 gene has a unique regulation of gene expression, and at the time of induction, colicin E1 protein is produced in very large amounts by the effect of the positive regulation as well. Furthermore, the colicin E1 protein has an antimicrobial function against *E. coli,* and can be regarded as a protein which is not required for the normal growth of the *E. coli* cells. In view of its nature, the expression of the colicin E1 gene during a normal period is strictly repressed. Hence, the utilization of the regulatory gene of the colicin E1 gene is very advantageous to the present invention.

Col E1 DNA can be obtained, for example, from Pharmacia P. L. Biochemicals, Inc. under Catalog No. 27-4914-01.

The base sequence of the regulatory gene of the colicin E1 gene consisting of a region participating in positive regulation, a promoter-operator region, and a ribosome binding region has already been reported (Y. Ebina, Gene, *15,* 119, 1981). The regulatory gene of the colicin E1 gene in the present invention contains the base sequence from No. −140 to No. 78 in Figure 1 as an essential segment.

In joining the human Cu, Zn-SOD cDNA to the downstream side of the regulatory gene of the colicin E1 gene, ligation in such a way as to produce so-called fused proteins can be relatively easily carried out by utilizing cleavage sites of restriction endonucleases. However, when a portion derived from the colicin E1 protein has a length larger than a certain limit, it is very probable that when administered to a human, the fused proteins will exhibit immunogenicity (antigenicity). Desirably, therefore, the N-terminus amino acid codon of the human Cu, Zn-SOD is joined immediately after the translation initiation codon (ATG) of the colicin E1 gene. However, neither of these genes has a suitable cleavage site of a restriction endonuclease which meets this desire. It is possible therefore to cut out both DNA fragments in a form having the required portion deleted by restriction endonucleases, and substitute a synthetic DNA fragment for the deleted portion.

The synthetic DNA fragment can be obtained by chemically synthesizing oligonucleotides by, for example, the solid phase triester method. (K. Miyoshi et al., Nucl. Acids Res., *8,* 5507, 1980) and ligating them with, for example, T4 DNA ligase. The synthetic DNA fragment does not necessarily have to reproduce the base sequence of the original deleted portion. It is well known that degeneracy exists in amino acid codons, and the frequency of codon usage differs from species to species of living organisms. Hence, so long as the amino acid sequence remain unchanged, any desired codons can be selected in the preparation of synthetic DNA fragments. If, however, codons having a high frequency of use in the host organism are selected, an increase in the expression of the gene can be expected. With regard to the regulatory gene region of the colicin E1 gene, it is preferred to reproduce the original base sequence in the synthetic DNA fragment, but a change in base sequence which will lead to an increase in the expression of the gene may be made.

The desired recombinant DNA can be obtained by correctly inserting a DNA fragment containing the regulatory gene of colicin E1 gene, a synthetic DNA fragment and a human Cu, Zn-SOD structural gene fragment into a vector capable of being autonomously replicated. These DNA fragments can be ligated, for example, by T4 DNA ligase. There is no restriction on the order of ligation of the DNA fragments so long as the structure of the finally obtained recombinant DNA is the desired one. Any vectors can be used which can be replicated in host microorganisms. When the host is *E. coli,* pBR322 is frequently used as the vector.

The resulting recombinant DNA is introduced into a host microorganism for the vector to transform the host. In the present invention. *E. coli* is used as the host microorganism, but other microorganisms such as *Bacillus subtilis* and *Saccharomyces cerevisiae* may also be used so long as the regulatory gene, particularly the regulatory gene of colicin E1 gene, can function. For example, W3110, 20S0 and C600S

strains of *E. coli* may be used. W3110 strain is especially preferred. W3110 strain is a $\lambda^-$, $F^-$ strain derived from a wild strain ($\lambda^+$, $F^+$) of *E. coli* K12 and is the same as the wild strain in auxotrophy, etc. (B. J. Bachmann, Bacteriological Reviews, *36*, 525, 1972).

Transformants are selected by, for example, tetracycline resistance, and in a customary manner, the plasmid DNA is separated. By analyzing the restriction map, the transformants are secondarily screened. The desired transformants are selected by determining the ability to produce human Cu, Zn-SOD during induction.

SOD activity may be measured, for example, by utilizing a method involving using cytochrome C/xanthine/xanthine oxidase (J. M. McCord and I. Fridovich, J. Biol. Chem., *244*, 6049), and a method involving using nitro blue tetrazolium (NBT)/rivoflavin (C. Beauchamp and I. Fridovich, Anal. Biochem., 10, *44*, 276, 1971). The NBT-rivoflavin method is simple and convenient because it can also be utilized in staining protein activities after electrophoresis. When *E. coli* is the host, human Cu, Zn-SOD produced from the recombinant DNA and Mn-SOD and Fe-SOD produced by the host chromosomes exist as a mixture in the transformants. The enzymatic actions of these SODs are the same but they can be distinguished from each other in that the activity of Cu, Zn-SOD is inhibited by 1—2 mM $CN^-$ ions whereas the activities of Mn-SOD and Fe-SOD are not inhibited under the same conditions. These three kinds of SOD differ from each other in molecular weight or isoelectric point, and therefore can be separated by electrophoresis, or ion exchange or gel filtration column chromatography.

The resulting microorganism transformed with the recombinant DNA having the human Cu, Zn-SOD structural gene downstream of the regulatory gene, particularly the regulatory gene of colicin E1 gene is grown for a predetermined period of time under conditions suitable for the growth of the host organism and then by induction, large quantities of human Cu, Zn-SOD are produced. *E. coli* transformants may be grown in a known culture medium such as L medium or M9 medium containing glucose and Casamino acid. The cultivation is carried out usually at a temperature of 15 to 43°C for a period of 2 to 24 hours, as required with aeration and agitation. Induction is carried out by adding an inducer, for example a drug such as mitomycin C or Nalidixic acid to the culture during the logarithmic growth period or applying ultraviolet irradiation to it.

After the induction, the cells are harvested and ruptured by known methods, and proteins having human Cu, Zn-SOD activity are purified from the cells by methods that are usually known. As a result, human Cu, Zn-SOD can be obtained. The purification can be carried out by, for example, heat-treatment, salting out, concentration, dialysis, ion-exchange chromatography, gel filtration chromatography, chromatofocusing, electrophoresis, high-performance liquid chromatography and affinity chromatography in suitable combinations.

The following Examples illustrate the present invention more specifically. Needless to say, the scope of the present invention is in no way limited to these Examples.

Example 1

Isolation of mRNA from human placenta

The human placenta obtained by normal parturition was minced, and rapidly frozen by liquid nitrogen. The frozen placenta was homogenized in the presence of liquid nitrogen to obtain a powdery placental tissue sample.

Five milliliters, per gram of the tissue sample, of a guanidinium solution (6 M guanidinium thiocyanate, 5 mM sodium citrate, 0.5% sodium N-lauroylsarcosinate, 0.1 M 2-mercaptoethanol) was added to the tissue sample, and the mixture was quickly homogenized by means of a disperser. The homogenate was passed three times through a 22G×1¼ syringe to shear the DNA. 5.7 M CsCl (containing 0.1 M EDTA, pH 7.5) was put in a centrifugal polyaromer tube) and two times its volume of the homogenate was overlaid on it. The homogenate was centrifuged in a rotor (Hitach RPS40T) at 25°C and 32,000 rpm for 17 hours. After the centrifugation, the supernatant was carefully removed, and the RNA precipitated at the bottom was washed with a small amount of ethanol to remove CsCl, and then dissolved in 1 ml of water. NaCl was added to the solution to a concentration of 0.2 M, and the solution was again precipitated with ethanol. The RNA was dissolved in 0.5 ml of 10 mM Tris-HCl (pH 7.5)+1 mM EDTA (to be refered to as TE), and mRNA was isolated by oligo(dT) cellulose (Type 3, Catalog No. 20003; Collaborative Research, Inc.).

The RNA solution was heated at 65°C for 5 minutes, and rapidly cooled. Then, 0.5 ml of 1 M NaCl was added and the solution was applied to an oligo (dT) cellulose column (bed volume 1.5 ml; column inside diameter 7 mm) equilibrated with TE+0.5 M NaCl. The column was washed with TE+0.5 M NaCl, and mRNA was eluted with TE. Fractions having an ultraviolet absorption at 254 nm were recovered, and NaCl was added to 0.2 M. mRNA was then precipitated with 2.5 volumes of ethanol. The precipitate was washed with 75% ethanol, and then lyophilized. From 5 g of the placental tissue sample, 50 micrograms of mRNA was obtained.

Example 2

Synthesis of cDNA

cDNA was synthesized in accordance with the method of Okayama-Berg (H. Okayama and P. Berg, Mol. Cell. Biol., *2*, 161, 1982).

Four hundred micrograms of pBR322-SV40 (0.71—0.86) DNA was digested with 700 units of *Kpn*I (a

product of Takara Shuzo Co., Ltd.; Catalog No. 1068A) at 37°C for 5 hours in 400 microliters of a buffer containing 6 mM Tris-HCl (pH 7.5), 6 mM $MgCl_2$, 6 mM NaCl, 6 mM 2-mercaptoethanol and 0.1 mg/ml of bovine serum albumin (BSA). The reaction was stopped by adding 40 microliters of 0.25 M sodium ethylenediaminetetraacetate (EDTA, pH 8.0) and 20 microliters of 10% sodium laurylsulfate (SDS). The reaction mixture was extracted with 0.4 ml of a water-saturated phenol/chloroform mixture (1:1). Then, by performing two ethanol precipitations and one washing with 70% ethanol, DNA was recovered.

The dT chain was added to the *Kpn*I cleavage terminal of the DNA with terminal transferase (TTase) in 200 microliters of a solution consisting of 140 mM sodium cacodylate, 30 mM Tris-HCl (pH 6.8), 1 mM $CoCl_2$, 0.1 mM dithiothreitol (DTT), and 0.25 mM dTTP [containing 0.5 µCi of ($\alpha-^{32}P$)]. Four hundred units of TTase (a product of Takara Shuzo Co., Ltd.; Catalog No. 2230B) was added to the reaction mixture kept at 37°C, the reaction was initiated. A small aliquot was removed from the reaction mixture at every predetermined time. The amount of $^{32}P$ taken up in the trichloroacetic acid (TCA) precipitate was measured, and the average chain length of the dT chain was determined. When the average chain length reached 60±10 bases, the reaction was stopped by adding 20 microliters of 0.25 M EDTA and 10 microliters of 10% SDS. Phenol-chloroform extraction was carried out four times, and then by the same treatment as in the preceding step, DNA was recovered. The average chain length of the dT chain in this example was 70 bases.

The DNA was digested with 17 units of *Hpa*I (a product of Takara Shuzo Co., Ltd.; Catalog No. 1064B) at 37°C for 5 hours in 200 liters of a buffer consisting of 10 mM Tris-HCl (pH 7.4), 10 mm $MgCl_2$, 20 mM KCl, 1 mM DTT and 0.1 mg/ml BSA. After stopping the reaction, the reaction mixture was subjected to 1% agarose gel electrophoresis, and a longer DNA fragment (about 2.7 kb) was separated. The DNA fragment was recovered from the gel by electrophoretic elution method (T. Maniatis et al., Molecular Cloning, 164, 1982, Cold Spring Harbor Laboratory), and then purified on oligo (dA) cellulose (a product of Collaborative Research, Inc. Catalog No. 20005) (bed volume 0.7 ml). The recovered peak fraction was used as a vector primer DNA.

Separately, 100 micrograms of pBR322-SV40 (0.19—0.32) DNA was digested with 120 units of *Pst*I (a product of Takara Shuzo Co., Ltd.; Catalog No. 1973A) at 37°C for 1.5 hours in 200 microliters of a buffer consisting of 6 mM Tris-HCl (pH 7.4), 6 mM $MgCl_2$, 6 mM 2-mercaptoethanol, 50 mM NaCl and 0.1 mg/ml BSA. The reaction was stopped and the reaction mixture was treated in the same way as above. The dG chain was added to the *Pst*I cleavage terminal of the DNA in the same way as in the case of adding the dT chain (including the after-treatment) except that 1 µCi of [$\alpha-^{32}P$]dGTP was used as a substrate and 60 units of TTase was used. The average chain length of the dG chain in this example was 27 bases. The DNA was then digested with 50 units of *Hin*dIII (a product of Takara Shuzo Co., Ltd.; Catalog No. 1060A) at 37°C for 1 hour, and a smaller DNA fragment (about 0.28 kb) was isolated and recovered by 1.8% agarose gel electrophoresis and used as a linker DNA. Incidentally, the vector primer DNA and the linker DNA above can also be obtained from Pharmacia P. L. Biochemicals (Catalogs No. 27-4948-01 and 27-4950-01).

Then, cDNA was synthesized in 20 microliters of a solution consisting of 50 mM Tris-HCl (pH 8.3), 8 mM $MgCl_2$, 30 mM KCl, 0.3 mM DTT, 2 mM dNTP (for each of 4 kinds), 10 µCi [$\alpha-^{32}P$] dCTP (3,000 Ci/mmole), 0.9 microgram mRNA, 1.4 micrograms vector primer DNA and 4 units of reverse transcriptase (a product of Life Science Co.). The reaction was carried out at 37°C for 30 minutes. $^{32}P$ was taken up in an amount corresponding to an average chain length of 1000 to 2000 bases. The reaction was stopped and the reaction mixture was after-treated in the same way as in the preceding step. The DNA was dried under reduced pressure, and then the dC chain was added. The DNA was dissolved in 15 microliters of a TTase reaction solution [140 mM sodium cacodylate, 30 mM Tris-HCl (pH 6.8), 1 mM 1 mM $CoCl_2$, 0.1 mM DTT, 0.2 microgram poly(A), and 66 M [$\alpha-^{32}P$]dCTP (10 µCi)]. Eighteen units of TTase was added, and the reaction was initiated at 37°C. When the average chain length of the dC chain became nearly equal to the dC chain of the linker DNA, the reaction was stopped, and the reaction mixture was worked up in the same way as in the preceding step. Ten microliters of the DNA was dissolved in a buffer for *Hin*dIII, and 2.5 units of *Hin*dIII was added. The mixture was reacted at 37°C for 1 hour. The reaction product was after-treated and then circularized by using the linker DNA.

The linker DNA was added to the DNA in an amount of 0.04 pmol for 0.02 pmole of the DNA. The mixture was heated in 10 microliters of TE+0.1 M NaCl at 65°C for 5 minutes, and then at 42°C for 30 minutes, and then cooled to 0°C. The resulting mixture was reacted overnight at 12°C in 100 microliters of a solution consisting of 20 mM Tris-HCl (pH 7.5), 4 mM $MgCl_2$, 10 mM $(NH_4)_2SO_4$, 0.1 M KCl, 0.1 mM beta-nicotinamide adenine dinucleotide (beta-NAD), 50 micrograms/ml of BSA and 0.6 microgram of *E. coli* DNA ligase (a product of Pharmacia P. L. Biochemicals, Inc., Catalog No. 27-0872-01). Furthermore, 40 M of each of four kinds of dNTP, 0.1 mM beta-NAD, 0.4 microgram of *E. coli* DNA ligase, 0.3 microgram of *E. coli* DNA polymerase (a product of Takara Shuzo Co., Ltd.; Catalog No. 2130A), 1 unit of *E. coli* RNase H (a product of Pharmacia P. L. Biochemicals, Inc.; Catalog No. 27-0894-01) were additionally supplied, and the volume of the reaction mixture was adjusted to 104 microliters. The reaction was carried out at 12°C for 1 hour, and then at 25°C for 1 hour.

Example 3

Preparation and transformation of competent cells

*E. coli* DHL strain was inoculated in 400 ml of an SOB medium consisting of 2% bacto-tryptone (a

product of Difco Laboratories), 0.5% bacto-yeast extract (a product of Difco Laboratories), 10 mM NaCl, 2.5 mM KCl, 10 mM $MgCl_2$ and 10 mM $MgSO_4$, and grown at 37°C with shaking until the $OD_{550}$ of the culture reached about 0.6. The culture was chilled on ice for 10 minutes, and then centrifuged at 4°C and 6,000 rpm for 5 minutes to harvest the cells. The cells were suspended in 140 ml of a Tfbl solution (30 mM $KOCOCH_3$, 100 mM RbCl, 10 mM $CaCl_2$, 50 mM $MnCl_2$, 15% glycerol; adjusted to pH 5.8 with 0.2 M acetic acid). The suspension was left to stand at 0°C for 5 minutes and then centrifuged to collect the cells. The cells were suspended in 32 ml of a TfbII solution (10 mM MOPS, 75 mM $CaCl_2$, 10 mM RbCl, 15% glycerol; adjusted to pH 6.5 with KOH). The suspension was divided into 200 microliter portions and instantaneously frozen in a dry ice-ethanol bath. The frozen products were stored at −80°C.

The frozen cell suspension was left to stand at room temperature, and when it was melted, left to stand at 0°C for 15 minutes. Twenty microliters of the DNA solution prepared in Example 12 was added, and the mixture was left to stand at 0°C for 30 minutes. Thermal shock was applied to the mixture by heating it at 42°C for 90 seconds. The mixture was then placed on an ice bath for 2 minutes, and 800 microliters of the SOB medium was added. The mixture was gently shaken at 37°C for 1 hour. The cells were then subjected to colony hybridization.

Example 4
Synthesis of synthetic DNA probes

As probes in colony hybridization, two oligonucleotides having the following sequences

5'TTCGTCGCCAT3' (undecamer)
5'TTATTGGGCGATCCCAATT3' (nonadecamer)

were synthesized. They had sequences complementary to the base sequences (the underlined portions in Figure 2) corresponding to the N-terminus and C-terminus of human Cu, Zn-SOD. The synthesis was carried out by the solid phase triester method. The undecamer probe was synthesized by successively coupling protected nucleotide dimer blocks CA, GC, TC, CG and TT (all products of Bachem Inc.) with 10 mg of a polystyrene support (a product of Wako Pure Chemicals, Co., Ltd.; Catalog No. 16011681). A reaction of removing the 5'-terminus protective group was carried out by using a 1 M solution of $ZnBr_2$ in dichloromethaneisopropanol (85:15). The coupling reaction was carried out at 45°C for 15 minutes in 0.15 ml of a pyridine solution containing 0.1 M dimer block and mesitylene sulfonyl-3-nitrotriazole in an amount of 3 equivalents to the dimer block. The unreacted reactive groups were blocked with acetic anhydride, and the next coupling reaction was carried out.

When the final coupling reaction was terminated, the mixture was reacted at 40°C for 20 hours in 3000 microliters of 0.5 M tetramethyl guanidium-2-pyridine aldoximate to cause the oligonucleotide to leave the polystyrene support. Protective groups other than the 5'-terminus protective group were removed by using 28% aqueous ammonia. Then, the product was subjected to high-performance liquid chromatography using a reverse phase column (a product of Toyo Soda Industry Co., Ltd.; ODS-120A, 4.6 mm in diameter×25 cm) using 0.5% ammonium formate buffer by the acetonitrile concentration gradient elution method. An oligonucleotide peak eluted at an acetonitrile concentration of about 30% was recovered, and reacted at 25°C for 30 minutes in 80% acetic acid to remove the 5'-terminus protective group. The product was again purified on a reverse phase column, and a peak eluted at an acetonitrile concentration of 17% was recovered and used as a probe for colony hybridization.

The nonadecamer probe was synthesized in the same manner as described above. The yield of the undecamer probe was 41%, and the yield of the nonadecamer probe was 6%.

Example 5
Selection of the recombinant DNA by colony hybridization

A sterilized nitrocellulose filter was placed on an L agar plate containing 100 micrograms/ml of ampicillin, and the cells obtained in Example 3 was spread on the filter and grown at 37°C. When the diameter of the colonies became about 0.1 mm, the colonies were transferred on two separate nitrocellulose filters. The filters were placed on an L-agar plate containing 250 micrograms/ml of chloramphenicol, and incubated at 37°C for 24 hours. The colonies on the filters were lysed with 0.5 M NaOH and neutralized, and then heated at 65°C under reduced pressure for 2 hours to fix the DNA on the filters. The filters were immersed in 500 ml of 5× Denhardt's solution having 0.5% SDS and 10 mM EDTA, and slowly shaken at 68°C for 6 hours. The filters were then immersed in a solution consisting of 5× SET, 5× Denhardt's solution, 0.1% SDS, 250 micrograms/ml of denatured *E. coli* DNA, and 10 micrograms/ml of $^{32}P$-labelled synthetic DNA probe to perform hybridization for 48 hours at 28°C for the undecamer probe and 40°C for the nonadecamer probe. The $^{32}P$-labelled probes were found by using T4 polynucleotide kinase (a product of Takara Shuzo Co., Ltd.; Catalog No. 2020A) to have a specific activity of $1.1\times10^9$ cpm/microgram and $9.4\times10^9$ cpm/microgram, respectively, the SET was composed of 0.15 M NaCl, 30 mM Tris-HCl (pH 8) and 1 mM EDTA, and the Denhardt's solution as composed of 0.02% Ficoll, 0.02% polyvinyl pyrrolidone and 0.02% BSA. The filters were washed, and autoradiographed. Colonies which gave positive signals to both of the probes were selected, and cultivated. The plasmid DNA was isolated and the base sequence of cDNA was examined by the Maxam-Gilbert method and the dideoxy method.

As a result, only one colony harboring plasmid pSOD2 having the whole region of the human Cu, Zn-SOD structural gene was obtained from more than 300 thousand colonies.

Figure 2 shows the base sequence of the human Cu, Zn-SOD structural gene and its amino acid sequence determined on the basis of the base sequence.

When the human Cu, Zn-SOD so obtained was compared with the human Cu, Zn-SOD which Sherman et al. (*supra*) isolated from an established cell line derived from patients with Down's syndrome, it was found that the codon for the 54th threonine is not ACG but ACA. But the amino acid sequence was the same for both of these human Cu, Zn-SOD genes. Jabush et al. (*supra*) reported the amino acid sequence of Cu, Zn-SOD derived from human erythrocytes. A comparison of the human Cu, Zn-SOD obtained in this example showed that the 17th amino acid is not SER but ILE, and the 98th amino acid is not VAl but SER. Elsewhere, the 26th amino acid is not ASP but ASN; the 49th amino acid is not GLN but GLU; the 52nd amino acid is not ASN but ASP; and the 53rd amino acid is not ASP but ASN. These four additional amino acids are difficult to identify because of the technical problems in amino acid analysis, and may be considered as substantially identical for the two human Cu, Zn-SOD genes.

It was therefore confirmed from the foregoing that the human Cu, Zn-SOD structural gene existed on plasmid pSOD2.

Example 6

Construction of recombinant DNA

(1) Isolation of the regulatory gene fragment of colicin E1 gene and its insertion into a vector

Ten micrograms of Col E1 DNA was digested with 8 units of *Dra*I (a product of Takara Shuzo Co., Ltd.: Catalog No. 1037) at 37°C for 12 hours in 50 microliters of a buffer consisting of 10 mM Tris-HCl (pH 7.5), 7 mM $MgCl_2$, 60 mM NaCl, and 7 mM 2-mercaptoethanol. DNA was recovered by ethanol precipitation, and then digested with 10 units of *Stu*I (a product of Nippon Gene Co., Ltd.) at 37°C for 12 hours in 50 microliters of a buffer consisting of 100 mM NaCl, 10 mM Tris-HCl (pH 7.5), 10 mM $MgCl_2$ and 6 mM 2-mercaptoethanol. The digestion product was subjected to 5% polyacrylamide gel electrophoresis, and a 340 bp *Stu*I-*Dra*I fragment was recovered by the electrophoretic elution method.

Separately, 10 micrograms of pBR322 DNA was digested with 13 units of *Dra*I at 37°C for 12 hours under the same conditions as used in the digestion of Col E1 DNA. The digestion product was subjected to 1.2% agarose gel electrophoresis, and a DNA fragment having a size of 3651 bp was recovered in the same way as above. This fragment was reacted with 0.2 unit of alkaline phosphatase (a product of Takara Shuzo Co., Ltd.; Catalog No. 2120A) at 25°C for 2 hours to remove the phosphate group at the 5'-terminus. DNA was recovered by phenol treatment, ether extraction and ethanol precipitation.

The 340 bp Col E1 fragment (0.1 microgram) and 2 micrograms of the alkaline phosphatase-treated 3651 bp pBR322 fragment were ligated with 1 unit of T4 DNA ligase at 15°C for 12 hours using a ligation buffer consiting of 66 mM Tris-HCl (pH 7.6), 6.6 mM $MgCl_2$, 10 mM DTT, 0.4 mM adenosine triphosphate (ATP).

The resulting DNA was introduced into *E. coli* DH1 strain in the same way as in Example 3. Tetracycline resistance transformants were selected, and the plasmid DNA was isolated. A plasmid pAOK 1 having the 340 bp Col E1 *Stu*I-*Dra*I fragment inserted into the 3651 bp *Dra*I bp fragment of pBR322 was obtained as a result of analysis of the restriction endonuclease cleavage map (see Figure 4). In the pAOK 1, transcription from the regulatory gene of colicin E1 gene was in a direction reverse to the transcription direction of the tetracycline-resistance gene.

(2) Isolation of the SOD structural gene fragment

Ten micrograms of pSOD 2 DNA was digested with 6 units of *Alu*I (a product of Takara Shuzo Co., Ltd.; Catalog No. 1004A) at 37°C for 12 hours in 50 microliters of a buffer consisting of 10 mM Tris-HCl (pH 7.5), 7 mM $MgCl_2$, 20 mM NaCl and 7 mM 2-mercaptoethanol. DNA was recovered by ethanol precipitation, and digested with 3 units of *Taq*I (a product of Takara Shuzo Co., Ltd.; Catalog No. 1092B) at 65°C for 12 hours in 50 ml of a buffer consisting of 10 mM Tris-HCl (pH 7.5), 10 mM $MgCl_2$, 100 mM NaCl and 10 mM 2-mercaptoethanol. The digestion product was subjected to 5% polyacrylamide gel electrophoresis, and a 440 bp fragment was recovered in the same way as in (1) above. The *Taq*I cleavage site existed in a codon corresponding to the 20th amino acid of SOD, i.e. phenylalanine, and the *Alu*I cleavage site existed in a 3' non-translational region.

(3) Preparation of a synthetic DNA fragment

The required portion having a size of 84 bp which was deleted in the 340 bp Col E1 fragment and SOD structural gene fragment was divided into 12 oligonucleotides as shown in Figure 3, and synthesized by the method shown in Example 4. The oligonucleotides were each labelled with $^{32}P$ using $[\gamma-^{32}P]$ATP and T4 polynucleotide kinase. The oligonucleotides 1 to 6 were mixed, and the oligonucleotides 7 to 12 were also mixed. They were ligated by T4 DNA ligase. Specifically, the oligonucleotides were mixed each in an amount of 40 pmoles. The mixture in the form of an aqueous solution was heated at 90°C for 3 minutes, and then rapidly cooled. The mixture was left to stand at 19°C for 8 hours after adding a buffer having the same composition as in the ligation shown in (1) above. Then, T4 DNA ligase was added, and the mixture was reacted at 19°C for 24 hours. The reaction product was subjected to 15% polyacrylamide gel

electrophoresis and then autoradiographed to detect DNA bands. A gel was sliced from the band having a size corresponding to the desired size. The gel slice was crushed, and 0.3 ml of TE buffer was added. The mixture was left to stand at 37°C for 12 hours to extract DNA from the gel. The DNA solution was centrifuged. The supernatant was recovered, precipitated with ethanol, and then dried under reduced pressure.

The two DNA fragments so obtained were dissolved in 15 microliters of water, and mixed. The mixture was heated at 45°C, then left to stand at 19°C for 2 hours, and ligated with T4 DNA ligase. The ligation product was worked up in the same way as above except that electrophoresis was carried out using 10% polyacrylamide gel. As a result, a synthetic DNA fragment having a size of 84 bp was obtained.

In the synthetic DNA fragment shown in Figure 3, a codon corresponding to the 12th amino acid, glycine, is changed from GGC used in the original cDNA to GGT, and the codon for the 15th glutamine is changed from CAG to CAA. The codons GGT and CAA are used in a host organism more frequently than the codons GGC and CAG.

(4) Construction of recombinant DNA

pAOK 1 DNA was cleaved with *Dra*I, and then treated with alkaline phosphatase. 0.38 pmole of this fragment, 0.56 pmole of the synthetic DNA fragment and 0.56 pmole of the SOD structural gene fragment were mixed, and ligated with 1 unit of T4 DNA ligase at 4°C for 2 hours and then at 15°C for 12 hours. Transformants were selected for tetracycline resistance, and cultured. The recombinant DNA was isolated. The resulting recombinant DNA was digested with *Taq*I, and then subjected to 5% polyacrylamide gel electrophoresis. The desired recombinant DNA was cleaved with *Taq*I into 8 fragments having a size of 1307, 1115, 498, 475, 368, 315, 312 and 141 bp. Two hundred transformants were examined, and recombinant DNAs pUBE1, pUBE2, pUBE3 and pUBE4 having the same electrophoretic pattern as the desired recombinant DNA were obtained.

Example 7

Induction of SOD synthesis

The four transformants harboring the recombinant DNAs obtained in Example 6 and a transformant harboring pAOK 1 was induced with mitomycin C, and their ability to produce human Cu, Zn-SOD was measured. The transformant harboring pAOK 1 was selected for tetracycline resistance after introduction of pAOK 1 DNA into W3110 in the same way as in Example 3, and used herein as a comparison to determine the amount of Mn-SOD and Fe-SOD produced from genes on the host chromosomes.

The transformants were each inoculated in 20 ml of M9 medium containing 0.3% Casamino acid (a product of Difco Laboratories), 0.4% glucose and 10 micrograms/ml of tetracycline, and grown with shaking at 37°C until the $OD_{660}$ of the culture reached 0.4. The culture was centrifuged at 4°C and 8,000 rpm for 10 minutes to harvest the cells. The cells were then suspended in 20 ml of a freshly prepared medium of the aforesaid composition, and mitomycin C (a product of Kyowa Fermentation Co., Ltd.) was added in a concentration of 2 micrograms/ml. The mixture was incubated at 37°C for 2 hours to induce synthesis of SOD.

The cells were collected by centrifugation at 4°C and 8,000 rpm for 10 minutes, washed with 10 ml of physiological saline, and suspended in 3 ml of physiological saline. The suspension was sonicated to rupture the cells, and centrifuged at 4°C and 12,000 rpm for 15 minutes. The supernatant crude extract was recovered, and its SOD activity was measured by the method of Beauchamp et al. (*supra*). Specifically, 10 microliters of the crude extract was added to 3 ml of a solution consisting of $5.6\times10^{-5}$ M NBT (a product of Wako Pure Chemicals, Co.; Catalog No. 148-01991), $1\times10^{-2}$ M methionine, $5.85\times10^{-6}$ M rivoflavin and 50 mM potassium phosphate buffer (pH 7.8). The mixture was irradiated with light from a 18 W fluorescent lamp for 2 minutes, and the absorbance (OD) at 560 nm of the mixture was measured. At the same time, the absorbance at 560 nm of a sample containing 10 microliters of water in place of the crude extract was also measured, and the ratio of inhibition of NBT reduction based on the degree of NBT reduction of this sample was measured. The ratio of inhibition of NBT reduction has a positive correlation with the SOD activity, and the higher the inhibition ratio, the higher the SOD activity. Furthermore, the above measurement was repeated except that 2 mM of KCN was further added. The effect of $CN^-$ ions was examined. The results are shown in Table 1.

TABLE 1

| Transformant | Protein concentration (mg/ml) | SOD activity | | | | | |
|---|---|---|---|---|---|---|---|
| | | In the absence of $CN^-$ | | | In the presence of $CN^-$ | | |
| | | $OD_{560}$ | % Inhibition of NBT reduction | Relative activity | $OD_{560}$ | % Inhibition of NBT reduction | Relative activity |
| W3110 (pAOK1) | 1.01 | 0.253 | % 40.5 | 1.00 | 0.326 | % 39.2 | 1.00 |
| W3110 (pUBE1) | 1.09 | 0.166 | 60.9 | 1.39 | 0.294 | 45.1 | 1.07 |
| W3110 (pUBE2) | 1.03 | 0.069 | 83.8 | 2.03 | 0.277 | 48.3 | 1.21 |
| W3110 (pUBE3) | 1.03 | 0.109 | 74.3 | 1.80 | 0.325 | 39.4 | 0.99 |
| W3110 (pUBE4) | 1.15 | 0.080 | 81.2 | 1.76 | 0.275 | 48.7 | 1.09 |
| When 10 microliters of water was added | | 0.425 | 0 | | 0.536 | 0 | |

In the above table, the protein concentration was measured by using a protein assay reagent (a product of Bio-Rad Laboratories; Catalog No. 500-0001) and bovine serum albumin (BSA) as a standard sample. The relative activity value is a value obtained by correcting the effect of the amount of protein.

The transformants harboring the recombinant DNAs showed 1.4 to 2 times as high SOD activity as W3110 (pAOK1). The increased activity was due to human Cu, Zn-SOD. This is clearly seen from the fact that in the presence of $CN^-$ ions, they showed nearly the same level of activity. The presence of $CN^-$ increased $OD_{560}$, but this effect can be ignored by comparing the inhibition ratios. *E. coli* W3110 (pUBE2) which showed the highest activity in this example was deposited in Fermentation Research Institute, Tsukuba, Japan under deposit number FERM BP-634 on October 12, 1984.

Example 8

The results of Example 7 demonstrate the production of human Cu, Zn-SOD, but a confirmatory test was further carried out. As a standard sample for comparison, Cu, Zn-SOD (a product of Sigma Chemical Co.; Catalog No. S7006) prepared from human erythrocytes was used. This standard sample had a specific activity of 3,000 units/mg of protein.

A crude extract of W3110 (pUBE2) was subjected to electrophoresis by the gel disc electrophoretic method ("Methods of Basic Experiments in Proteins and Enzymes", edited by Horio and Yamashita, 282, 1981, published by Nankodo), and the gel after electrophoresis was stained. The acrylamide concentration was 15% for a separated gel and 0.3% for a concentrated gel, and the gels were prepared by chemical polymerization using N,N,N',N'-tetramethylethylenediamine (TEMED). A Tris-glycine (pH 8.3) buffer was used for the electrophoresis.

The gel after the electrophoresis was immersed for 20 minutes in a $2.45\times10^{-3}$ M NBT solution in a light shielded condition, and then immersed for 15 minutes in a solution consisting of 0.028 M TEMED, $2.8\times10^{-5}$ M rivoflavin and 0.036 M potassium phosphate buffer (pH 7.8). Then, light was irradiated onto the gel for 8 minutes using a 18 W fluorescent lamp. By the light irradiation, the gel turned from transparent yellow to semi-transparent dark bluish violet, but a band of SOD protein remains uncolored and looked transparent. 0.38 microgram of SOD of Sigma Chemical Co. and the same amount of the crude extract of W3110 (pUBE2) containing human Cu, Zn-SOD (the total amount of proteins was 131 micrograms) showed protein bands having the same mobility and the same transparency with no color change, and it could be confirmed that both are substantially the same. 164 micrograms of the crude extract of W3110 (pAOK1) was also subjected to electrophoresis. But no band having SOD activity was detected and it was found that under these conditions, no detectable amounts of Mn-SOD and FeO-SOD were produced.

Next, activity inhibition by an anti-human Cu, Zn-SOD antibody (to be referred to simply as the antibody hereinafter) was examined. 1.5 mg of human Cu, Zn-SOD (a product of Sigma Chemical Co.) was subcutaneously injected to rabbits together with Freund's complete adjuvant. The same injection was given 16 days and 18 days later. Six days after the final immunization, the blood was drawn from the animals, and the antisera were separated. After heat-treatment at 56°C for 30 minutes, IgG was isolated by chromatography on a column of protein A-Sepharose Cl-4B (Pharmacia P. L. Biochemicals, Inc.; Catalog No. 17-0780-01). The bed volume was 2 ml, and the column was eluted with 1 M acetic acid. The eluate was assayed by the Ouchterlony method. As a result, the isolated IgG formed precipitation arcs with human Cu, Zn-SOD (Sigma Chemical Co.), but showed no precipitation arcs with Cu, Zn-SOD (a product of Sigma Chemical Co.; Catalog No. 58254) derived from bovine erythrocytes and the crude extract of W3110. It was determined therefore that the isolated IgG was an antibody specific to human Cu, Zn-SOD.

Fifty microliters of the antibody was added to 10 microliters each of the crude extracts of W3110 (pUBE2) and W3110 (pAOK1), and reacted at 4°C for 4 hours. The same reaction was carried out except that 50 microliters of physiological saline was added instead of the antibody. After the reaction, the activity was measured in the same way as in Example 7. The results are shown in Table 2.

TABLE 2

| Sample | Antibody | OD at 560 nm | Difference of OD values (added)—(none) |
|---|---|---|---|
| Crude extract of W3110 | None<br>Added | 0.278<br>0.250 | 0.002 |
| Crude extract of W3110 (pUBE2) | None<br>Added | 0.158<br>0.246 | 0.088 |
| Human Cu, Zn-SOD (Sigma Chemical Co.) | None<br>Added | 0.66<br>0.417 | 0.251 |
| Not added | None<br>Added | 0.420<br>0.423 | 0.003 |

W3110 (pAOK1) which produced only Mn-SOD and Fe-SOD did not change in the degree of NBT reduction by the addition of the antibody, whereas W3110 (pUBE2) increased in the degree of NBT reduction by the addition of the antibody and its degree of NBT reduction became nearly equal to that of W3110 (pAOK1). This is because only the effect of inhibiting NBT reduction by human Cu, Zn-SOD was cancelled by the addition of the antibody.

The foregoing results led to the confirmation that the transformant W3110 (pUBE2) harboring recombinant DNA pBUE2 produced human Cu, Zn-SOD. In the next example, human Cu, Zn-SOD was produced by using these transformants.

Example 9

Production of human Cu, Zn-SOD

W3110 (pUBE2) was inoculated in 5 liters of M9 medium containing 0.3% Casamino acid, 0.4% glucose and 10 micrograms ml tetracycline, and grown with shaking at 37°C until the $OD_{660}$ of the culture reached 0.4. Then, in the same way as in Example 7, induction was carried out at 37°C for 2 hours. Centrifugation gave 20 g of wet cells. The cells were washed with physiological saline and then suspended in 5 mM Tris-HCl (pH 7.5). The suspension was sonicated and then centrifuged at 14,000 rpm for 15 minutes at 4°C to give 155 ml of a crude extract.

The crude extract was heat-treated at 60°C for 3 minutes. The resulting precipitate was removed by centrifugation at 14,000 ppm for 15 minutes at 4°C to obtain 142 ml of the supernatant. Streptomycin sulfate was added to the supernatant to a concentration of 2.5% (W/V). The resulting precipitate was centrifuged in the same manner as above to obtain 140 ml of the supernatant. The supernatant was precipitated with ammonium sulfate. Fractions precipitated with 50—90% saturated ammonium sulfate were collected, dialyzed and chromatographed on an ion exchange cellulose DE52 (a product of Whatman Ltd.) column (bed volume 43.3 ml; inside column diameter 2.1 cm). The column was eluted with NaCl having a concentration gradient from 0 to 0.2 M. The elution pattern of the SOD activity is shown in Figure 5. Three SOD activity peaks were detected, and the second peak fraction whose activity disappeared in the presence of 2 mM KCN was recovered.

The activity measurement shown in Figure 5 was for the purpose of detecting the peak fractions. Hence, a considerably large amount of SOD was added, and the accuracy of quantification is low. It is to be noted therefore that the ratio of the SOD peak areas in Figure 5 does not exactly represent the ratio of the total activities of the three SODs.

The second peak fraction was concentrated by ultrafiltration, and chromatographed on a column of Sephacryl S-200 (a product of Pharmacia Fine Chemicals, Co.; Catalog No. 17-0871-01) (bed volume 315 ml; inside column diameter 2.1 cm) to recover an active fraction of human Cu, Zn-SOD. This fraction was lyophilized. The results of purification are shown in Table 3.

TABLE 3

| Sample | Total activity (units) | Proteins (mg) | Specific activity (units/mg) | Percent recovery of activity (%) |
|---|---|---|---|---|
| Crude extract | 25.4×$10^2$ | 906.8 | 28 | — |
| Purified SOD | 9.4×$10^2$ | 2.3 | 4090 | 37.0 |

Purified human Cu, Zn-SOD having a specific activity of 4090 units/mg of protein was obtained in a recovery ratio of 37.0%.

Example 10
Production in a jar fermentor

One hundred liters of the same culture medium as in Example 9 was charged into a 200-liter jar fermentor equipped with stirring vanes. W3110 (pUBE2) was inoculated in the medium, and grown at 37°C and 200 rpm with aeration and agitation until the $OD_{660}$ of the culture reached 0.4. Then, 2 liters of an aqueous solution containing 30% $Na_2HPO_4$, 15% $KH_2PO_4$, 2.5% NaCl and 5% $NH_4Cl$, 1 liter of a 40% aqueous solution of glucose and 500 ml of a 0.04% aqueous solution of mitomycin C were added to the culture for induction, and the cells were further incubated at 37°C for 2 hours. The culture was centrifuged at 20,000 for 3 hours in a sharpless-type centrifuge to harvest 344 g of wet cells.

The cells were suspended in 3 liters of 10 mM triethanolamine buffer (pH 7.0) and the cells were ruptured by a Dyno-mill, followed by centrifugation at 9,000 rpm for 60 minutes. The supernatant was recovered, and a part of it was subjected to 12.5% SDS-polyacrylamide gel electrophoresis. The protein bands were stained with Coomassie Brilliant Blue. The density of each of the protein bands was measured by a densitometer. It was found that the proportion of human Cu, Zn-SOD in the entire proteins was 12%. For identification of the band of the human Cu, Zn-SOD protein, the purified SOD obtained in Example 9 was used.

**Claims**

1. A recombinant DNA comprising a regulatory gene having a positive regulation site and a human Cu-Zn superoxide dismutase structural cDNA said positive regulation site being a specific site to bind a complex of cyclic AMP and cyclic AMP receptor protein, and the codon for the N-terminus alanine of said human Cu-Zn superoxide dismutase structural gene existing immediately after the translation initiation codon of the regulatory gene which is the regulatory gene of colicin E1 gene.

2. The recombinant DNA of claim 1 wherein the human Cu-Zn superoxide dismutase structural cDNA is a complementary DNA derived from messenger RNA isolated from normal human tissues.

3. The recombinant DNA of claim 1 wherein the human Cu-Zn superoxide dismutase structural cDNA is a complementary DNA derived from messenger RNA isolated from the human placenta.

4. The recombinant DNA of claim 1 wherein the human Cu-Zn superoxide dismutase structural cDNA is a complementary DNA derived from messenger RNA separated from normal human tissues, and at least one of the codons is replaced by codons which are frequently used in a host organism so long as the replacement does not alter the amino acid sequence coded by the cDNA.

5. The recombinant DNA of claim 4 wherein the host organism is *Escherichia coli.*

6. The recombinant DNA of claim 1 wherein the human Cu-Zn superoxide dismutase structural cDNA has the following base sequence:

(5′) GCG ACG AAG GCC GTG TGC GTG CTG
AAG GGC GAC GGT CCA GTG CAA GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA
CTG GTG GTC CAT GAA AAA GCA GAT GAC
TTG GGC AAA GGT GGA AAT GAA GAA AGT

ACA AAG ACA GGA AAC GCT GGA AGT CGT
TTG GCT TGT GGT GTA ATT GGG ATC GCC
CAA (3').

7. The recombinant DNA of claim 1 wherein the human Cu-Zn superoxide dismutase structural cDNA has the following base sequence:

(5') GCG ACG AAG GCC GTG TGC GTG CTG
AAG GGC GAC GGC CCA GTG CAG GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA
CTG GTG GTC CAT GAA AAA GCA GAT GAC
TTG GGC AAA GGT GGA AAT GAA GAA AGT
ACA AAG ACA GGA AAC GCT GGA AGT CGT
TTG GCT TGT GGT GTA ATT GGG ATC GCC
CAA (3').

8. *Escherichia coli* transformed with the recombinant DNA of claim 1.

9. A process for producing human Cu-Zn superoxide dismutase, which comprises cultivating *Escherichia coli* transformed with the recombinant DNA of claim 1, and recovering the human Cu-Zn superoxide dismutase accumulated in the culture.

**Patentansprüche**

1. Rekombinante DNA, dadurch gekennzeichnet, daß sie ein Regulationsgen mit einer positiven Regulationsstelle und eine humane Cu-Zn-Superoxid-Dismutase-Struktur-cDNA enthält, wobei die positive Regulationsstelle eine spezifische Stelle ist, um einen Komplex von cyclischem AMP- und cyclischem AMP-Rezeptor-Protein zu binden, und das Codon für das N-Terminus-Alanin des humanen Cu-Zn-Superoxid-Dismutase-Strukturgens unmittelbar nach dem Translations-Initiations-Codon des Regulationsgens vorhanden ist, welches das Regulationsgen von Colicin-E1-Gen ist.

2. Rekombinante DNA nach Anspruch 1, dadurch gekennzeichnet, daß die humane Cu-Zn-Superoxid-Dismutase-Struktur-cDNA eine Komplementär-DNA ist, die sich von Messenger-RNA ableitet, die aus normalen humanen Geweben isoliert wurde.

3. Rekombinante DNA nach Anspruch 1, dadurch gekennzeichnet, daß die humane Cu-Zn-Superoxid-Dismutase-Struktur-cDNA eine Komplementär-DNA ist, die sich von Messenger-RNA ableitet, die aus humaner Placenta isoliert wurde.

4. Rekombinante DNA nach Anspruch 1, dadurch gekennzeichnet, daß die humane Cu-Zn-Superoxid-Dismutase-Struktur-cDNA eine Komplementär-DNA ist, die sich von Messenger-RNA ableitet, die aus normalen humanen Geweben abgetrennt wurde, und daß mindestens eines der Codons durch Codons ersetzt ist, welche häufig in Wirtsorganismen verwendet werden, solange der Ersatz die Aminosäure-Sequenz, die durch cDNA codiert wird, nicht ändert.

5. Rekombinante DNA nach Anspruch 4, dadurch gekennzeichnet, daß der Wirtsorganismus *Escherichia coli* ist.

6. Rekombinante DNA nach Anspruch 1, dadurch gekennzeichnet, daß die humane Cu-Zn-Superoxid-Dismutase-Struktur-cDNA die folgende Basensequenz aufweist:

(5') GCG ACG AAG GCC GTG TGC GTG CTG
AAG GGC GAC GGT CCA GTG CAA GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA
CTG GTG GTC CAT GAA AAA GCA GAT GAC

TTG GGC AAA GGT GGA AAT GAA GAA AGT
ACA AAG ACA GGA AAC GCT GGA AGT CGT
TTG GCT TGT GGT GTA ATT GGG ATC GCC
CAA (3').

7. Rekombinante DNA nach Anspruch 1, dadurch gekennzeichnet, daß die humane Cu-Zn-Superoxid-Dismutase-Struktur-cDNA die folgende Basensequenz aufweist:

(5') GCG ACG AAG GCC GTG TGC GTG CTG
AAG GGC GAC GGC CCA GTG CAG GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA
CTG GTG GTC CAT GAA AAA GCA GAT GAC
TTG GGC AAA GGT GGA AAT GAA GAA AGT
ACA AAG ACA GGA AAC GCT GGA AGT CGT
TTG GCT TGT GGT GTA ATT GGG ATC GCC
CAA (3').

8. *Escherichia coil,* dadurch gekennzeichnet, daß es mit der rekombinanten DNA von Anspruch 1 transformiert ist.

9. Verfahren zur Herstellung von humaner Cu-Zn-Superoxid-Dismutase, dadurch gekennzeichnet, daß *Escherichia coli,* transformiert mit der rekombinanten DNA von Anspruch 1, gezüchtet wird und daß die humane Cu-Zn-Superoxid-Dismutase, die sich in der Kultur ansammelt, gewonnen wird.

**Revendications**

1. ADN recombinant comprenant un gène régulateur ayant un site de régulation positif et un ADNc de structure de la superoxyde dismutase Cu-Zn humaine, ledit site de régulation positif étant un site spécifique pour la fixation d'un complexe d'AMP cyclique et d'une protéine réceptrice d'AMP cyclique, et le codon pour l'alanine N-terminale dudit gène de structure de la superoxyde dismutase Cu-Zn humaine se trouvant juste après le codon d'initiation de traduction du gène régulateur qui est le gène régulateur du gène de la colicine E1.

2. ADN recombinant selon la revendication 1 dans lequel l'ADNc de structure de la superoxyde dismutase Cu-Zn humaine est un ADN complémentaire dérivant d'un ARN messager isolé de tissus humains normaux.

3. ADN recombinant selon la revendication 1 dans lequel l'ADNc de structure de la superoxyde dismutase Cu-Zn humaine est un ADN complémentaire dérivant d'un ARN messager isolé du placenta humain.

4. ADN recombinant selon la revendication 1 dans lequel l'ADNc de structure de la superoxyde dismutase Cu-Zn humaine est un ADN complémentaire dérivant d'un ARN messager séparé de tissus humains normaux, et au moins un des codons est remplacé par des codons qui sont souvent utilisés dans un organisme hôte à la conditions que le remplacement n'altère pas la séquence d'amino-acides codée par l'ADNc.

5. ADN recombinant selon la revendication 4 dans lequel l'organisme hôte est *Escherichia coli.*

6. ADN recombinant selon la revendication 1 dans lequel l'ADNc de structure de la superoxyde dismutase Cu-Zn humaine a la séquence de bases suivante:

(5') GCG ACG AAG GCC GTG TGC GTG CTG
AAG GGC GAC GGT CCA GTG CAA GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA

CTG GTG GTC CAT GAA AAA GCA GAT GAC
TTG GGC AAA GGT GGA AAT GAA GAA AGT
ACA AAG ACA GGA AAC GCT GGA AGT CGT
TTG GCT TGT GGT GTA ATT GGG ATC GCC
CAA (3').

7. ADN recombinant selon la revendication 1 dans lequel l'ADNc de structure de la superoxyde dismutase Cu-Zn humaine a la séquence de bases suivante:

(5') GCG ACG AAG GCC GTG TGC GTG CTG
AAG GGC GAC GGC CCA GTG CAG GGC ATC
ATC AAT TTC GAG CAG AAG GAA AGT AAT
GGA CCA GTG AAG GTG TGG GGA AGC ATT
AAA GGA CTG ACT GAA GGC CTG CAT GGA
TTC CAT GTT CAT GAG TTT GGA GAT AAT
ACA GCA GGC TGT ACC AGT GCA GGT CCT
CAC TTT AAT CCT CTA TCC AGA AAA CAC
GGT GGG CCA AAG GAT GAA GAG AGG CAT
GTT GGA GAC TTG GGC AAT GTG ACT GCT
GAC AAA GAT GGT GTG GCC GAT GTG TCT
ATT GAA GAT TCT GTG ATC TCA CTC TCA
GGA GAC CAT TGC ATC ATT GGC CGC ACA
CTG GTG GTC CAT GAA AAA GCA GAT GAC
TTG GGC AAA GGT GGA AAT GAA GAA AGT
ACA AAG ACA GGA AAC GCT GGA AGT CGT
TTG GCT TGT GGT GTA ATT GGG ATC GCC
CAA (3').

8. *Escherichia coli* transformé avec l'ADN recombinant de la revendication 1.

9. Procédé de production de superoxyde dismutase Cu-Zn humaine, procédé selon lequel on cultive *Escherichia coli* transformé avec l'ADN recombinant de la revendication 1, et on récupère la superoxyde dismutase Cu-Zn humaine accumulée dans la culture.

## Fig. 1

```
5' CCGCGCGCATATCACGGTAAAACTGTGAACGCGATCTGCCTGTCATTTTTAGTGCGTCCC
   GGCGCGCGTATAGTGCCATTTTGACACTTGCGCTAGACGGACAGTAAAAATCACGCAGGG

   GGAGTGTGTGCCATGCCATAAAGTGACAGTGTCCCATAGATGTCTCATCTCATAGTTTCA
   CCTCACACACGGTACGGTATTTCACTGTCACAGGGTATCTACAGAGTAGAGTATCAAAGT

  -160                -140                -120
   GTAAAACATAATGAGGTCTGAGAACGGTAATGTTTGTGCTGGTTTTTGTGGCATCGGGCG
   CATTTTGTATTACTCCAGACTCTTGCCATTACAAACACGACCAAAAACACCGTAGCCCGC

  -100      -90       -80       -70       -60       -50
   AGAATAGCGCGTGGTGTGAAAGACTGTTTTTTTGATCGTTTTCACAAAAATGGAAGTCCA
   TCTTATCGCGCACCACACTTTCTGACAAAAAAACTAGCAAAAGTGTTTTTACCTTCAGGT

  -40       -30       -20       -10    mRNA •——————→ 10
   CAGTCTTGACAGGGAAAATGCAGCGGCGTAGCTTTTATGCTGTATATAAAACCAGTGGTT
   GTCAGAACTGTCCCTTTTACGTCGCCGCATCGAAAATACGACATATATTTTGGTCACCAA
        RS                        PB
                                        DraI
   20        30        40        50    ↓    60        70
   ATATGTACAGTATTTATTTTTAACTTATTGTTTTAAAAGTCAAAGAGGATTTTATAATGG
   TATACATGTCATAAATAAAAATTGAATAACAAAATTTTCAGTTTCTCCTAAAATATTACC
                                                            Met
```

# Fig. 2

```
     1   2   3   4   5   6   7   8   9   10  11  12  13  14  15  16  17  18  19  20  21  22  23  24  25  26
    ALA THR LYS ALA VAL CYS VAL LEU LYS GLY ASP GLY PRO VAL GLN GLY ILE ILE ASN PHE GLU GLN LYS GLU SER ASN
ATG,GCG,ACG,AAG,GCC,GTG,TGC,GTG,CTG,AAG,GGC,GAC,GGC,CCA,GTG,CAG,GGC,ATC,ATC,AAT,TTC,GAG,CAG,AAG,GAA,AGT,AAT,

27  28  29  30  31  32  33  34  35  36  37  38  39  40  41  42  43  44  45  46  47  48  49  50  51  52  53
GLY PRO VAL LYS VAL TRP GLY SER ILE LYS GLY LEU THR GLU GLY LEU HIS GLY PHE HIS VAL HIS GLU PHE GLY ASP ASN
GGA,CCA,GTG,AAG,GTG,TGG,GGA,AGC,ATT,AAA,GGA,CTG,ACT,GAA,GGC,CTG,CAT,GGA,TTC,CAT,GTT,CAT,GAG,TTT,GGA,GAT,AAT,

54  55  56  57  58  59  60  61  62  63  64  65  66  67  68  69  70  71  72  73  74  75  76  77  78  79  80
THR ALA GLY CYS THR SER ALA GLY PRO HIS PHE ASN PRO LEU SER ARG LYS HIS GLY GLY PRO LYS ASP GLU GLU ARG HIS
ACA,GCA,GGC,TGT,ACC,AGT,GCA,GGT,CCT,CAC,TTT,AAT,CCT,CTA,TCC,AGA,AAA,CAC,GGT,GGG,CCA,AAG,GAT,GAA,GAG,AGG,CAT,

81  82  83  84  85  86  87  88  89  90  91  92  93  94  95  96  97  98  99  100 101 102 103 104 105 106 107
VAL GLY ASP LEU GLY ASN VAL THR ALA ASP LYS ASP GLY VAL ALA ASP VAL SER ILE GLU ASP SER VAL ILE SER LEU SER
GTT,GGA,GAC,TTG,GGC,AAT,GTG,ACT,GCT,GAC,AAA,GAT,GGT,GTG,GCC,GAT,GTG,TCT,ATT,GAA,GAT,TCT,GTG,ATC,TCA,CTC,TCA,

108 109 110 111 112 113 114 115 116 117 118 119 120 121 122 123 124 125 126 127 128 129 130 131 132 133 134
GLY ASP NIS CYS ILE ILE CLY ARG THR LEU VAL VAL HIS GLU LYS ALA ASP ASP LEU GLY LYS GLY GLY ASN GLU GLU SER
GGA,GAC,CAT,TGC,ATC,ATT,GGC,CGC,ACA,CTG,GTG,GTC,CAT,GAA,AAA,GCA,GAT,GAC,TTG,GGC,AAA,GGT,GGA,AAT,GAA,GAA,AGT,

135 136 137 138 139 140 141 142 143 144 145 146 147 148 149 150 151 152 153
THR LYS THR GLY ASN ALA GLY SER ARG LEU ALA CYS GLY VAL ILE GLY ILE ALA GLN STOP
ACA,AAG,ACA,GGA,AAC,GCT,GGA,AGT,CGT,TTG,GCT,TGT,GGT,GTA,ATT,GGG,ATC,GCC,CAA, TAA,
```

Fig. 3

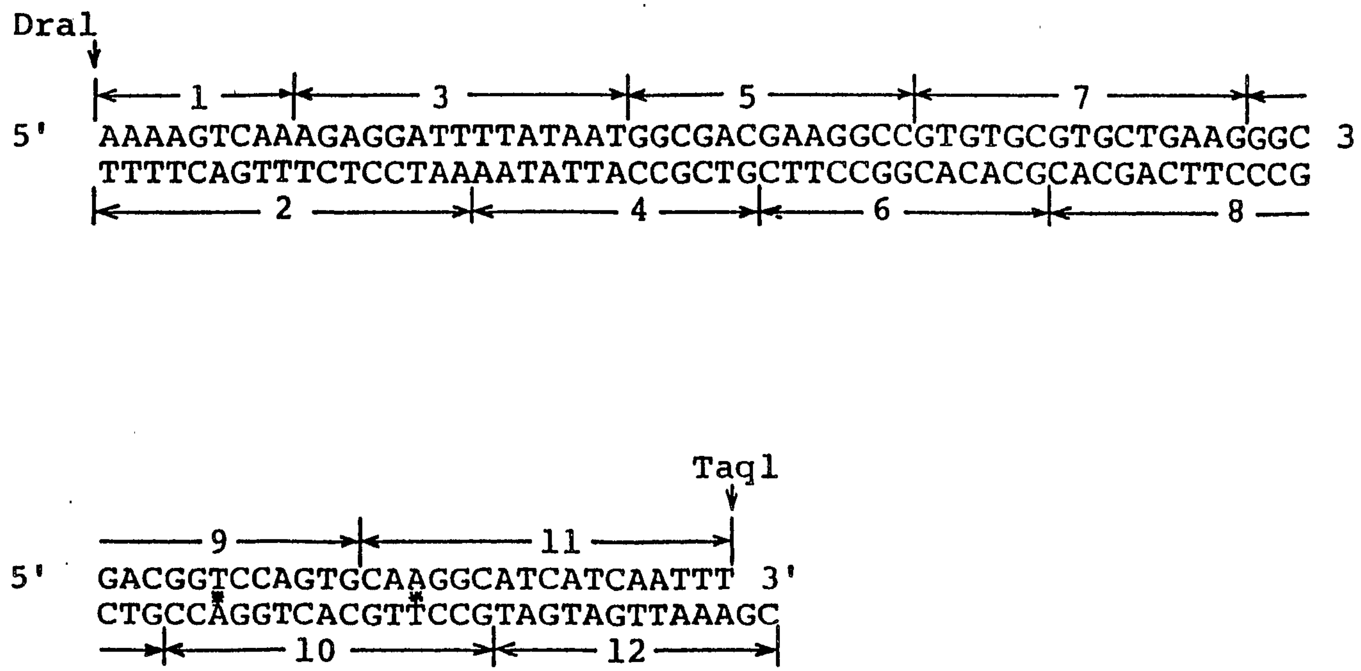
DraI
1
3
5
7
5' AAAAGTCAAAGAGGATTTTATAATGGCGACGAAGGCCGTGTGCGTGCTGAAGGGC 3'
TTTTCAGTTTCTCCTAAAATATTACCGCTGCTTCCGGCACACGCACGACTTCCCG
2
4
6
8
TaqI
9
11
5' GACGGTCCAGTGCAAGGCATCATCAATTT 3'
CTGCCAGGTCACGTTCCGTAGTAGTTAAAGC
10
12

*Fig. 4*

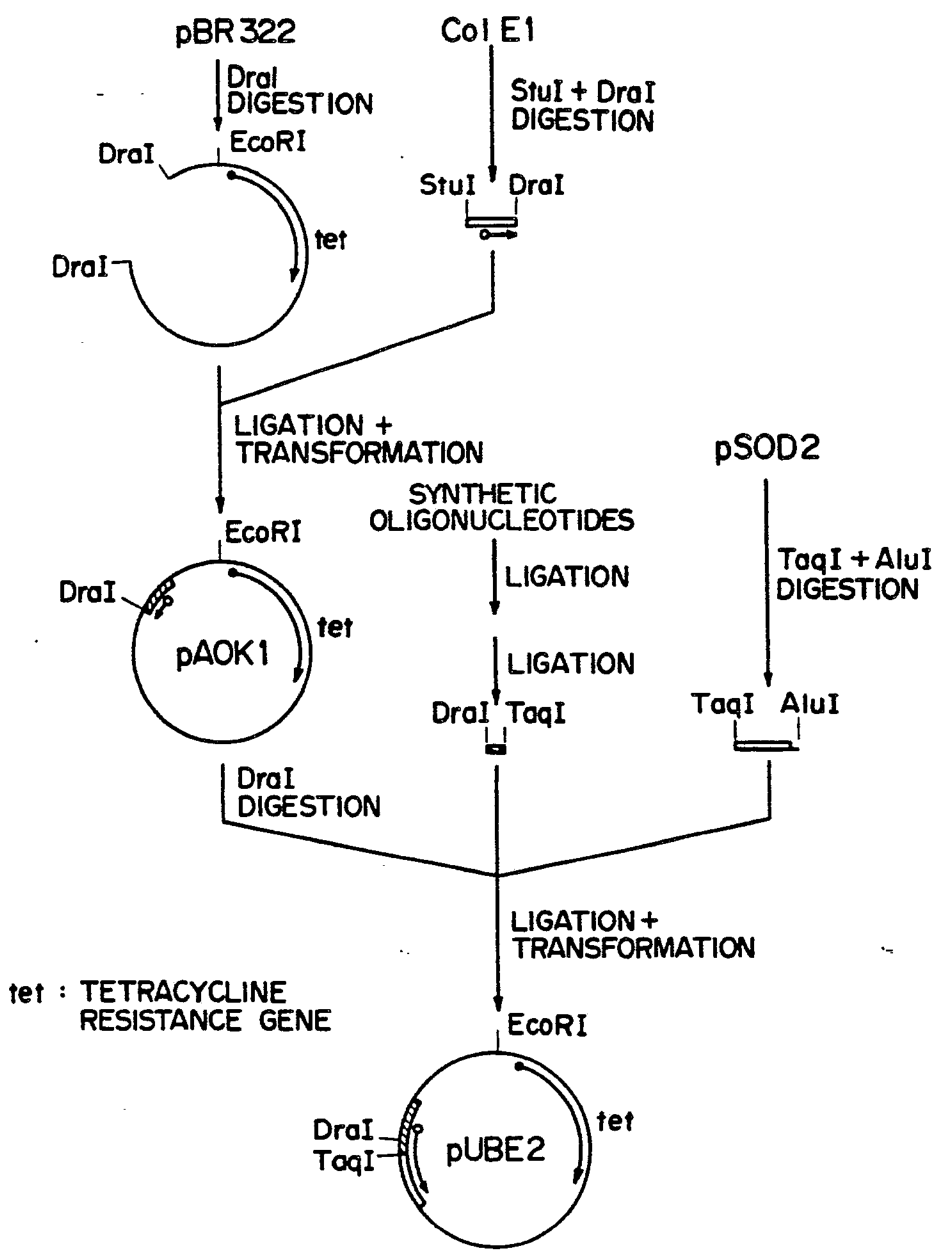
pBR322
DraI
DIGESTION
EcoRI
DraI
tet
DraI
Col E1
StuI + DraI
DIGESTION
StuI
DraI
LIGATION +
TRANSFORMATION
pSOD2
SYNTHETIC
OLIGONUCLEOTIDES
EcoRI
LIGATION
TaqI + AluI
DIGESTION
DraI
tet
pAOK1
LIGATION
DraI
TaqI
TaqI
AluI
DraI
DIGESTION
LIGATION +
TRANSFORMATION
tet : TETRACYCLINE
RESISTANCE GENE
EcoRI
tet
DraI
TaqI
pUBE2

*Fig. 5*

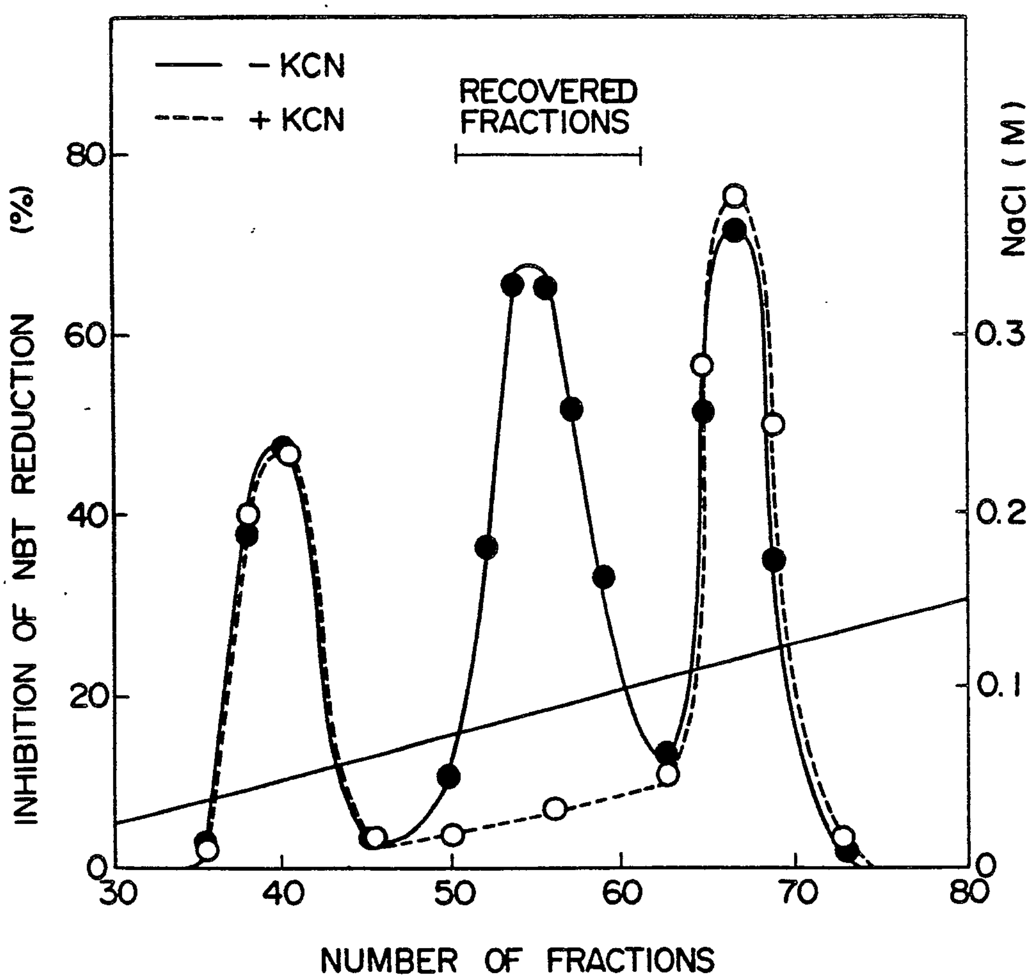
— KCN
+ KCN
RECOVERED FRACTIONS
INHIBITION OF NBT REDUCTION (%)
NaCl (M)
0
20
40
60
80
0.1
0.2
0.3
30
40
50
60
70
80
NUMBER OF FRACTIONS